# EUROPEAN PATENT APPLICATION

(11) **EP 4 431 078 A1**
(43) Date of publication of application: **18.09.2024**
(21) Application number: 22891229.1
(22) Date of filing: 11.11.2022
(51) Int. Cl.: A61J 1/05, A61J 1/14, B65D 47/36, B65D 41/50, A61M 39/00, B65D 47/38, B65D 41/34, A61J 1/20, B65D 5/06, B65D 47/32, B65D 41/20

(54) **FIXED CLOSURE FOR LONG-LIFE PACKAGE WITH AN ACCESS FOR ENTERAL NUTRITION EQUIPMENT FOR USE BY A CLOSED SYSTEM**

(30) Priority: 13.11.2021 BR 102021022914
(71) Applicant: Santos Leite, Ronaldo, 88190-000 Governador Celso Ramos/SC (BR)
(72) Inventor: Santos Leite, Ronaldo, 88190-000 Governador Celso Ramos/SC (BR)
(74) Representative: Cyrson, Matthew Dominic
(86) International application number: PCT/BR2022/050434
(87) International publication number: WO 2023/081991

(57) **Abstract**

The present invention falls within the technical field of Food Packaging and has been specifically created to cater to the needs of Enteral Nutrition Foods Administered by a Closed System, defining itself as an opening device that is part of an aseptic carton packaging for exclusive use through connection to the enteral feeding set. Its design redefines traditional aseptic carton packaging (used in open enteral systems) for a closed enteral system packaging, as its features allow for the infusion of the product through the enteral feeding tube and preserve the packaging's barriers and airtightness without exposing the product to the environment, eliminating handling and the consequent need for a specific preparation area.

## Description

### Field of the Invention

The present patent application belongs to the technical field of food packaging. It introduces an opening device for aseptic carton packaging, which, by replacing the traditional opening devices available in this type of packaging, allows it to be used for products exclusively intended for administration in a closed enteral system.

### Background of the Invention

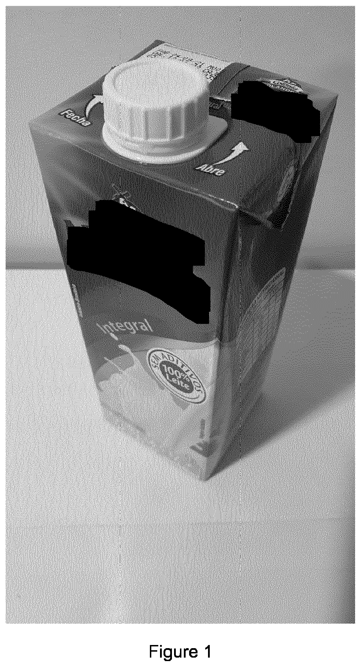

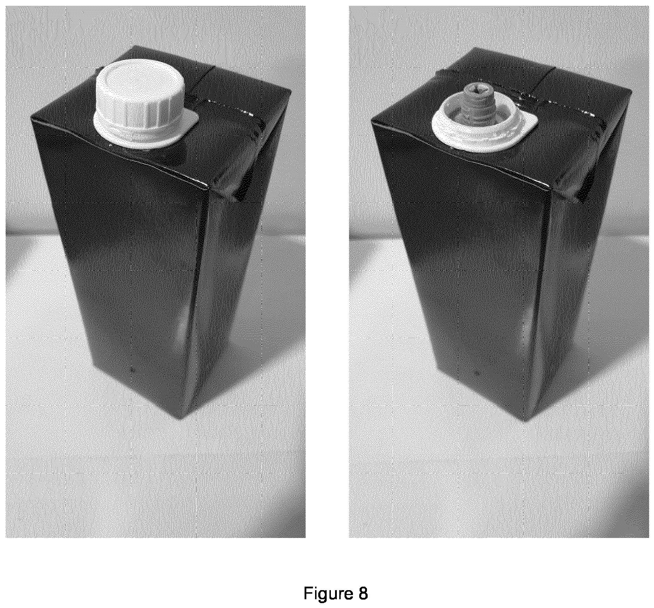

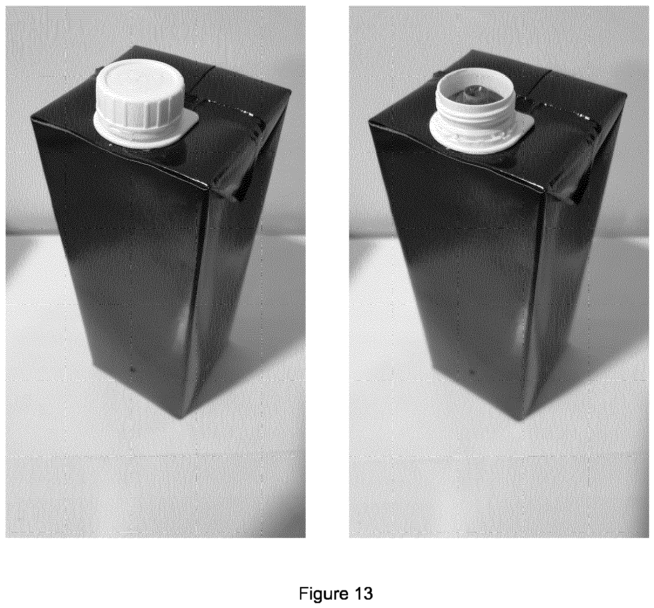

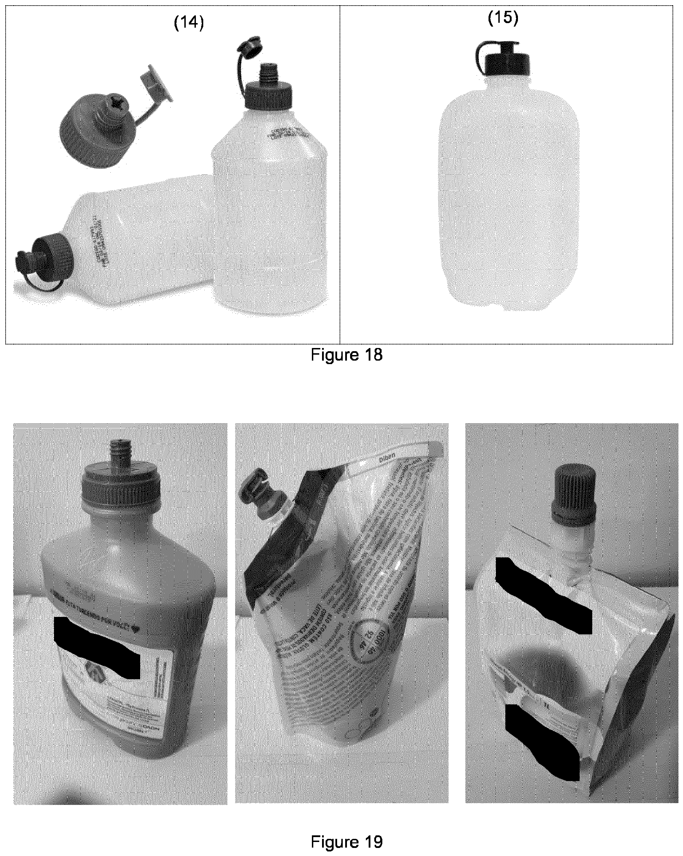

Enteral Nutritional Therapy can be carried out using two systems: open and closed. In the open enteral system, diets require handling and need to be prepared in specific areas. Meanwhile, the closed enteral system does not require specialized preparation or packaging areas. Foods for Enteral Nutrition are available in both systems. Open-system products, which are significantly cheaper, are supplied in aseptic carton packaging. Closed-system products are offered in various types of specially developed packaging (Figure 19). This invention transforms current aseptic carton packaging (Figure 1) into closed-system packaging (Figures 8 and 13), at a significantly lower cost, offering greater safety by eliminating the need for handling and providing superior quality compared to current closed-system products, which are typically sterilized by autoclaving.

Enteral Nutritional Therapy (ENT) involves high treatment costs for individuals, hospitals, health plans, or individuals treated in their own homes (HomeCare). If administration through a Closed System is recommended, this expense can triple compared to the open system. This characteristic implies that packaging specially developed for the closed system (Figure 19) significantly impacts the final product price, making it unaffordable for financially disadvantaged patients.

Currently, enteral formulas in aseptic carton packaging have automatic opening devices that require breaking the protective seal to access the product, thus excluding them from being classified as closed systems packaging . Breaking this seal exposes the contents to potential contamination from various sources, including manual contact or the atmosphere.

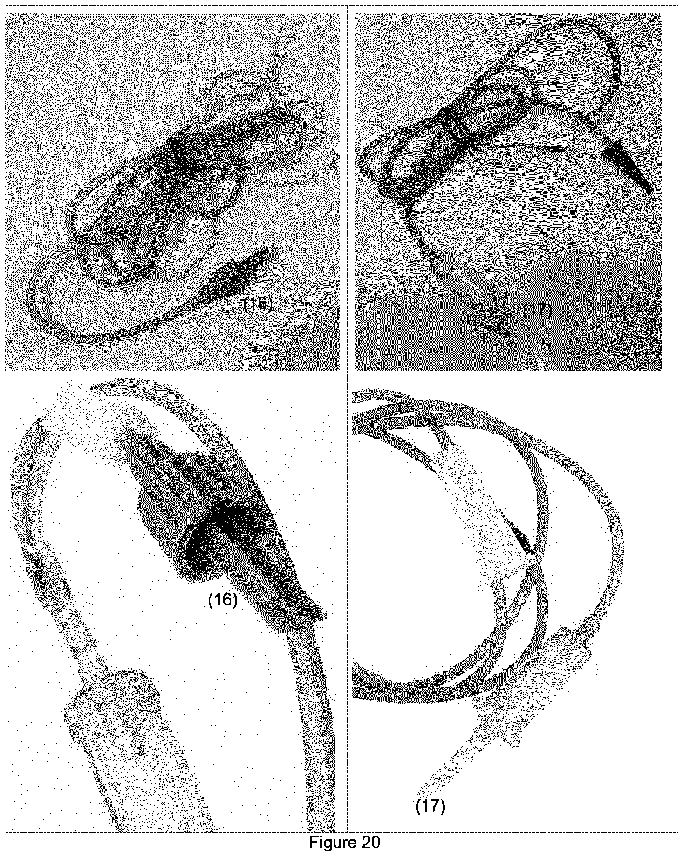

The present invention ensures the protective seal is breached appropriately because it is only perforated by the enteral feeding set (Figure 20), which is necessary for infusing Enteral Nutrition via the enteral set, preserving the contents inside the package similarly to special packaging used in closed-system products. Therefore, with aseptic carton packaging equipped with this invention, it is possible to drastically reduce the cost of Enteral Nutrition Foods for use in the Closed System and, consequently, the final consumer price.

In addition to the reduced price, another consistent technical advantage is maintaining the nutritional characteristics of the packaged product. The sterilization systems predominantly used in special packaging (autoclaving process) are considerably more thermally aggressive than those used in the long-life packaging process (UHT process), preserving the more natural state of the nutrients and reducing unwanted molecular interactions caused by sterilization. In other words, the invention reduces the price and enables a quality in the closed system equivalent to that found in the aseptic carton packaging open system.

### Description of the Drawings

The invention can take on various layouts with alternative mechanisms and complementary components. Below is a description of the figures provided to illustrate the invention and some of its concepts:
Figure 1 shows a photograph of traditional aseptic carton packaging.
Figure 2 shows a photograph of the prototype with a closed cap, the same for all layouts (except for the attached cap models represented in Figures 10 and 17).
Figure 3 shows a schematic drawing of the prototype with an access point for a cross-shaped enteral feeding set (ENFit^{®}), presented with the cap closed, with the upper part over the lower part.
Figure 4 shows a photograph of the prototype with an access point for a cross-shaped enteral feeding set (ENFit), with its upper (Cap) and lower (Base) parts separated and placed side by side.
Figure 5 shows a schematic drawing of the previous photograph (Figure 4), that is, of the prototype with its upper and lower parts separated.
Figure 6 represents the bottom view (2-a) and top view (2-b) of the base (02) of the opening device with an access point for a cross-shaped enteral feeding set and a biological filter.
Figure 7 represents the bottom view (2-a) and top view (2-b) of the base (02) of the opening device with an access point for a cross-shaped enteral feeding set without a biological filter.
Figure 8 shows photographs of the prototype with an access point for a cross-shaped enteral feeding set applied to an aseptic carton package, with the cap both closed and open.
Figure 9 shows a schematic drawing of the prototype with an access point for a cross-shaped enteral feeding set, differing in the fixation of the cap to the base using threads (08) and (09).
Figure 10 shows a schematic drawing of the prototype with an access point for a cross-shaped enteral feeding set, differing in the type of closure, which is achieved by pressing the cap onto the junction point.
Figure 11 shows a photograph of the prototype with an access point for a lancet-shaped enteral feeding set, with its upper and lower parts separated.
Figure 12 shows a schematic drawing of the previous photograph (Figure 11), that is, of the prototype with its upper and lower parts.
Figure 13 shows photographs of the prototype with an access point for a lancet-shaped enteral feeding set applied to an aseptic carton package, with the cap closed and with the cap open.
Figure 14 represents the bottom view (2-a) and top view (2-b) of the base (02) of the opening device with an access point for a lancet-shaped enteral feeding set with a biological filter.
Figure 15 represents the bottom view (2-a) and top view (2-b) of the base (02) of the opening device with an access point for a lancet-shaped enteral feeding set without a biological filter.
Figure 16 shows the schematic drawing of the prototype with an access point for a lancet-shaped enteral feeding set, differing in the fixation of the upper part to the lower part of the cap using partial threads (08) and (09).
Figure 17 shows a schematic drawing of the prototype with an access point for a lancet-shaped enteral feeding set, differing in the cap closure method, which is done by pressing the upper part (coupled via a foldable connection) over the lower part.
Figure 18 shows photographs illustrating two models of bottles used to administer open-system products with access points for cross-shaped (14) and lancet-shaped (15) enteral feeding sets.
Figure 19 shows photographs illustrating some models of special packaging used for closed-system products currently available on the market.
Figure 20 shows a photograph illustrating two feeding sets, cross-shaped (16) and lancet-shaped (17).

The components present in all layouts of the invention are described below:
(01) - Cap (upper part) of the opening device
(02) - Base (lower part) of the opening device
(03) - Tamper-evident seal (red dashed line)
(04) - Male thread of the internal device for securing a cross-shaped feeding set
(05) - Point for the penetration of the cross-shaped feeding set
(06) - Biological filter for internal decompression
(07) - Female thread compatible with male thread (04)
(08) - Male thread for securing the upper part
(09) - Female thread compatible with female part (08)
(10) - Upper part (cap) of the opening device coupled by a folding joint
(11) - Folding joint that attaches the upper and lower parts of the opening device
(12) - Point for penetration and securing of the lancet-shaped feeding set
(13) - Device for closing point for penetration (12)

### Description of the Invention

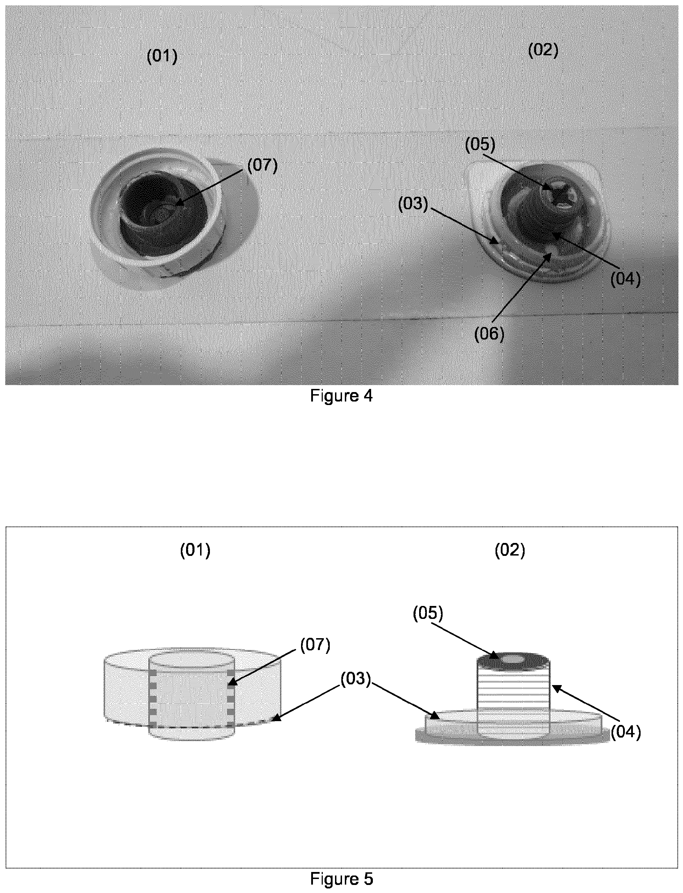

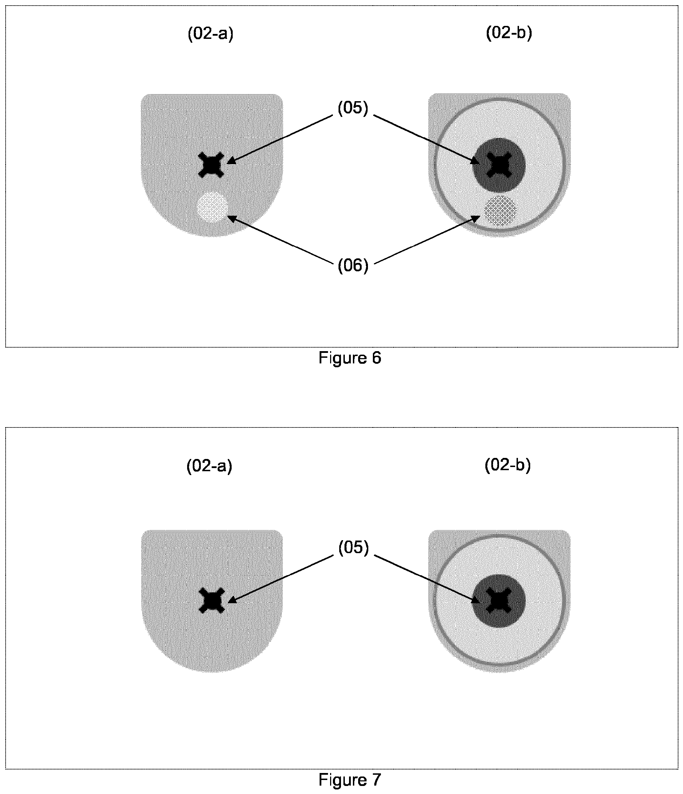

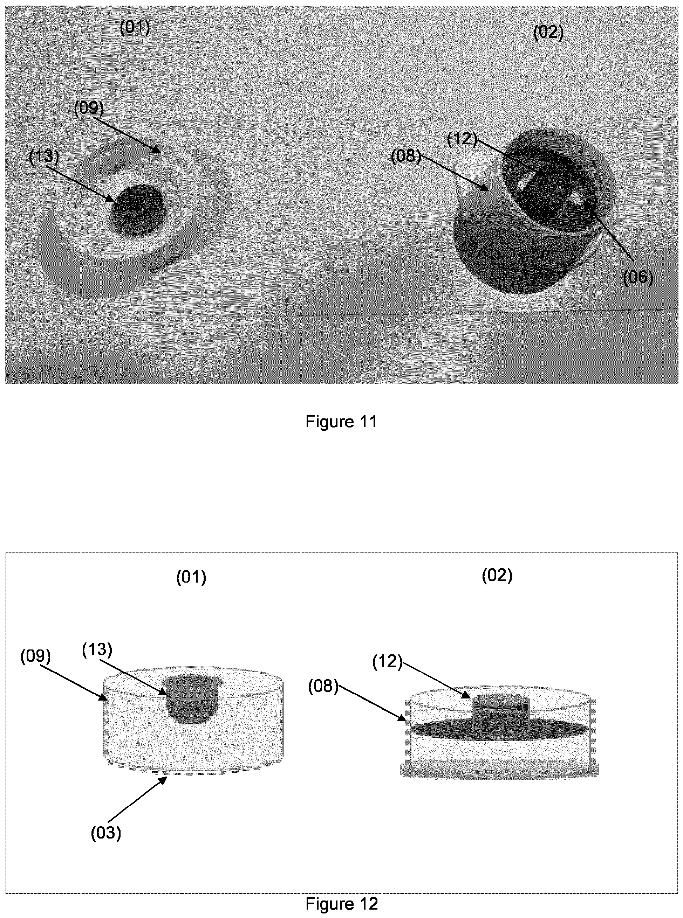

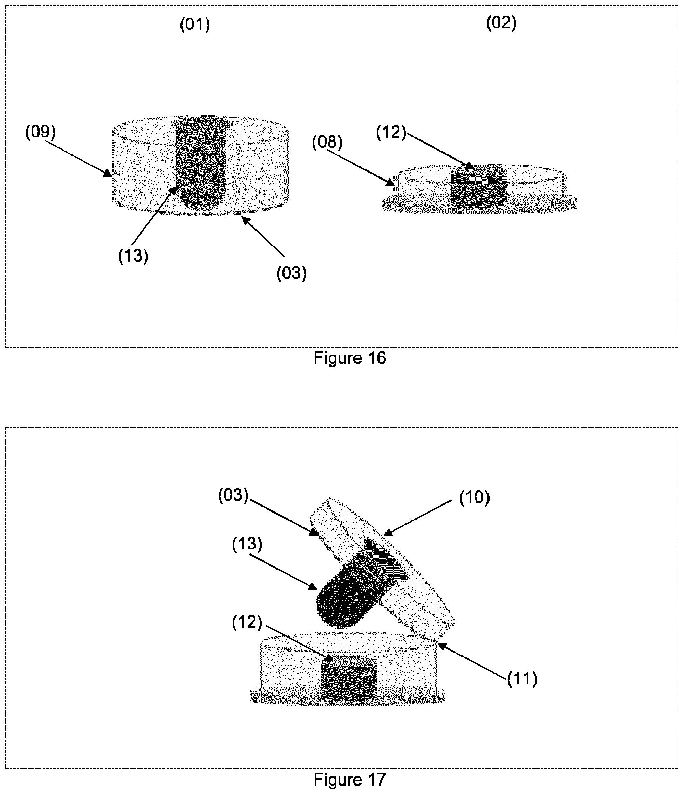

This opening device new will be an integral part of the aseptic carton packaging and, as shown in the figures explained above, can be realized in various designs. These designs may fit different types of feeding sets available on the market, streamline the production model, or improve handling through safer, more practical models that ensure product quality during administration. To illustrate compatibility with different models, the prototypes were presented for two different types of feeding sets: cross-shaped feeding set (figures 5, 9, 10) and lancet-shaped feeding set (Figures 12, 16, and 17). For these two types of feeding sets, the implementation options illustrated through different layouts can be applied with or without a biological filter, as shown in Figures 6, 7, 14, and 15.

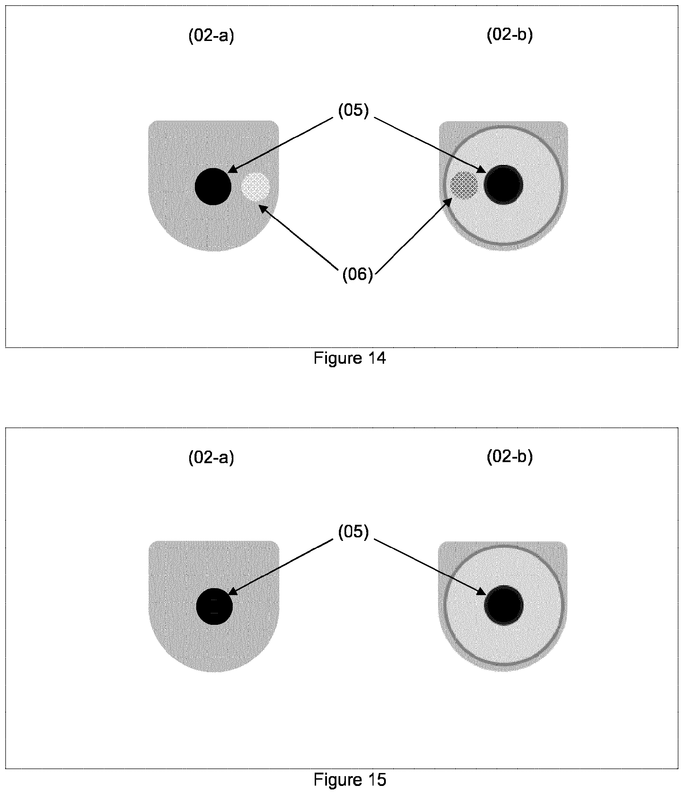

This invention, an opening device for aseptic carton packaging, regardless of the layout, will be exclusively part of carton packaging for products destined for enteral nutrition administration via a closed system. It includes features common to specialized packaging: an access point for feeding sets (Figure 20), whether cross-shaped (16), lancet-shaped (17), or other existing types; and a microbiological filter to facilitate the infusion of product through the enteral system (Figures 6 and 14) (06), allowing filtered air to enter and balance the internal pressure of the packaging. The microbiological filter is unnecessary in collapsible packages (Figures 7 and 15), which yield to pressure while maintaining satisfactory flow during product administration. This is why we present illustrative diagrams with filters (Figures 6 and 14) and without filters (Figures 7 and 15) for both cross-shaped access models (Figures 6 and 7) and lancet-shaped access models (Figures 14 and 15).

The inventive concept is illustrated by the photograph of the prototype (Figure 4) and its schematic drawing (Figure 5), depicting an opening device for the aseptic carton packaging with an access point for a cross-shaped feeding set at its base. It consists of a base (02) and a cap (01) featuring a female thread (07) compatible with the male thread (04) found at the base for securing the feeding set. The base is connected to the cap by the tamper-evident seal (03), which is broken when the cap (01) is removed to connect the enteral feeding set.

### Examples of Embodiments of the Invention

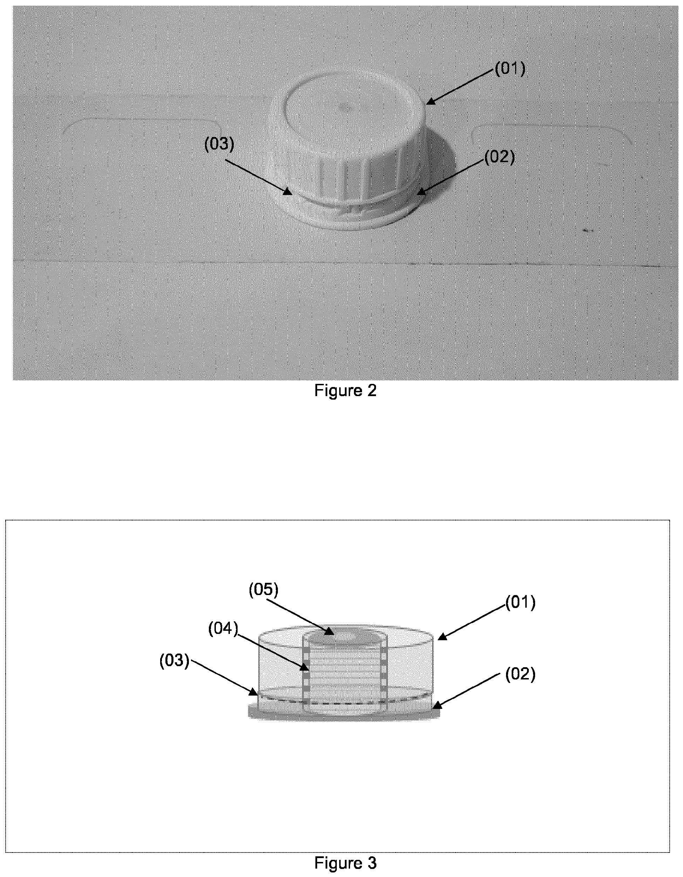

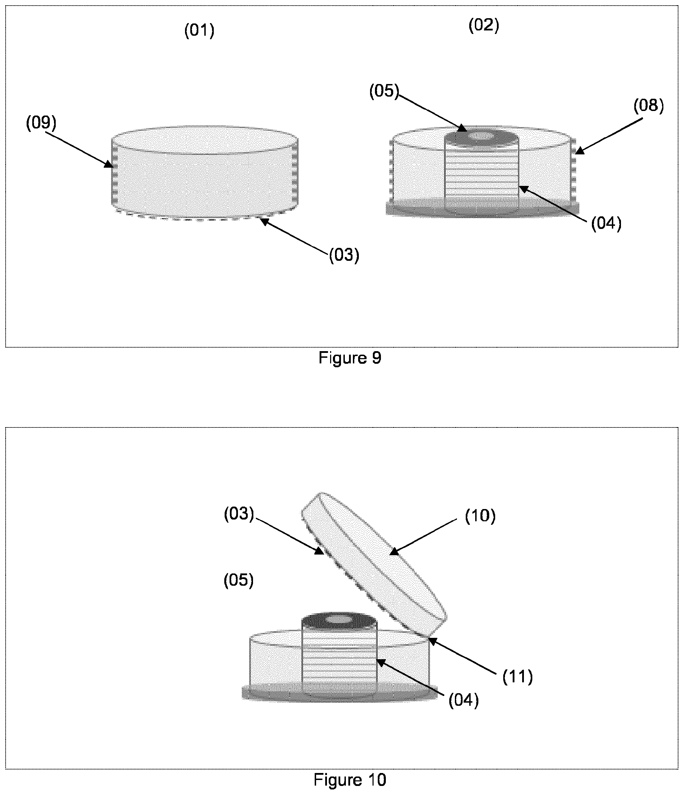

In addition to the proposal described above, some examples of embodiments of the invention are represented in Figures 9, 10, 12, 16, and 17. However, due to the ease of production and machinability for application in aseptic carton packaging, the form described in the previous paragraph may be the preferred one to be initially produced. Its model, mechanisms, and components can be understood by analyzing Figures 2, 3, 4, 5, 6, and 8.

Most food products in aseptic carton packaging have opening devices. However, the models used so far in this packaging do not have devices for securing an enteral feeding set. Therefore, this invention represents a novel solution for the enteral nutrition market, as it allows aseptic carton packaging to be used for foods in a closed enteral system.

## Claims

1. OPENING DEVICE FOR ASEPTIC CARTON PACKAGING USED FOR ENTERAL NUTRITION VIA CLOSED SYSTEM: **Characterized by** transforming aseptic carton packages into enteral nutrition food packages for feeding tube use in a closed system with a cross-shaped feeding set (Figure 5). It has a central thread (07) on the upper part (01) that fits into the internal male thread (04) of the internal device for fixing the cross-shaped feeding set, containing a biological filter (06) at its base (Figure 6).

2. OPENING DEVICE FOR ASEPTIC CARTON PACKAGING USED FOR ENTERAL NUTRITION VIA CLOSED SYSTEM, according to claim 1, **characterized by** transforming aseptic carton packages into enteral nutrition food packages for feeding tube use in a closed system with a cross-shaped feeding set (Figure 5). It has a central thread (07) on the upper part (01) that fits into the internal male thread (04) of the internal device for fixing the feeding set, without a biological filter at its base (Figure 7).

3. OPENING DEVICE FOR ASEPTIC CARTON PACKAGING USED FOR ENTERAL NUTRITION VIA CLOSED SYSTEM, according to claim 1, **characterized by** transforming aseptic carton packages into enteral nutrition food packages for feeding tube use in a closed system with a cross-shaped feeding set (Figure 9). It has a separate distal female thread (09) on the upper part (01) that fits into the male thread (08) of the lower part (02), containing a biological filter (06) at its base (Figure 6).

4. OPENING DEVICE FOR ASEPTIC CARTON PACKAGING USED FOR ENTERAL NUTRITION VIA CLOSED SYSTEM, according to claim 3, **characterized by** transforming aseptic carton packages into enteral nutrition food packages for feeding tube use in a closed system with a cross-shaped feeding set (Figure 9). It has a separate distal female thread (09) on the upper part (01) that fits into the male thread (08) of the lower part (02), without a biological filter at its base (Figure 7).

5. OPENING DEVICE FOR ASEPTIC CARTON PACKAGING USED FOR ENTERAL NUTRITION VIA CLOSED SYSTEM, according to claim 1, **characterized by** transforming aseptic carton packages into enteral nutrition food packages for feeding tube use in a closed system with a cross-shaped feeding set (Figure 10). The upper and lower parts are connected by a folding joint (11) that, even after the tamper-evident seal (03) is removed, allows opening without separating the parts and closing by pressing the upper part (10) onto the lower part. It has a biological filter (06) at its base (Figure 6).

6. OPENING DEVICE FOR ASEPTIC CARTON PACKAGING USED FOR ENTERAL NUTRITION VIA CLOSED SYSTEM, according to claim 5, **characterized by** transforming aseptic carton packages into enteral nutrition food packages for feeding tube use in a closed system with a cross-shaped feeding set (Figure 10). The upper and lower parts are connected by a folding joint (11) that, even after the tamper-evident seal (03) is removed, allows opening without separating the parts and closing by pressing the upper part (10) onto the lower part, without a biological filter at its base (Figure 7).

7. OPENING DEVICE FOR ASEPTIC CARTON PACKAGING USED FOR ENTERAL NUTRITION VIA CLOSED SYSTEM, according to claim 3, **characterized by** transforming aseptic carton packages into enteral nutrition food packages for feeding tube use in a closed system with a lancet-type feeding set (Figure 12). It has a separate distal female thread (09) on the upper part (01) that fits into the male thread (08) of the lower part (02), containing on the upper part a device (13) to close the fitting (12), which is positioned in the middle part of the base without direct contact with the package, with a biological filter (06) at its base (Figure 14).

8. OPENING DEVICE FOR ASEPTIC CARTON PACKAGING USED FOR ENTERAL NUTRITION VIA CLOSED SYSTEM, according to claim 7, **characterized by** transforming aseptic carton packages into enteral nutrition food packages for feeding tube use in a closed system with a lancet-type feeding set (Figure 12). It has a separate distal female thread (09) on the upper part (01) that fits into the male thread (08) of the lower part (02), containing on the upper part a device (13) to close the fitting (12), which is positioned in the middle part of the base without direct contact with the package, without a biological filter at its base (Figure 15).

9. OPENING DEVICE FOR ASEPTIC CARTON PACKAGING USED FOR ENTERAL NUTRITION VIA CLOSED SYSTEM, according to claim 7, **characterized by** transforming aseptic carton packages into enteral nutrition food packages for feeding tube use in a closed system with a lancet-type feeding set (Figure 16). It has a separate partial distal female thread (09) on the upper part (01) that fits into the male thread (08) of the lower part (02), containing on the upper part an extended device (13) to close the fitting (12), which is positioned on the lower part of the base in direct contact with the package, with a biological filter (06) at its base (Figure 14).

10. OPENING DEVICE FOR ASEPTIC CARTON PACKAGING USED FOR ENTERAL NUTRITION VIA CLOSED SYSTEM, according to claim 9, **characterized by** transforming aseptic carton packages into enteral nutrition food packages for feeding tube use in a closed system with a lancet-type feeding set (Figure 16). It has a separate partial distal female thread (09) on the upper part (01) that fits into the male thread (08) of the lower part (02), containing on the upper part an extended device (13) to close the fitting (12), which is positioned on the lower part of the base in direct contact with the package, without a biological filter at its base (Figure 15).

11. OPENING DEVICE FOR ASEPTIC CARTON PACKAGING USED FOR ENTERAL NUTRITION VIA CLOSED SYSTEM, according to claim 5, **characterized by** transforming aseptic carton packages into enteral nutrition food packages for feeding tube use in a closed system with a lancet-type feeding set (Figure 17). The upper and lower parts are connected by a folding joint (11) that, even after the tamper-evident seal (03) is removed, allows opening without separating the parts and closing by pressing the upper part (10) onto the lower part. The upper part contains an extended device (13) to close the fitting (12), and it has a biological filter (06) at its base (Figure 14).

12. OPENING DEVICE FOR ASEPTIC CARTON PACKAGING USED FOR ENTERAL NUTRITION VIA CLOSED SYSTEM, according to claim 11, **characterized by** transforming aseptic carton packages into enteral nutrition food packages for feeding tube use in a closed system with a lancet-type feeding set (Figure 17). The upper and lower parts are connected by a folding joint (11) that, even after the tamper-evident seal (03) is removed, allows opening without separating the parts and closing by pressing the upper part (10) onto the lower part. The upper part contains an extended device (13) to close the fitting (12), without a biological filter at its base (Figure 15).
